# EUROPEAN PATENT APPLICATION

(11) **EP 3 059 686 A2**
(43) Date of publication of application: **24.08.2016**
(21) Application number: 16155652.7
(22) Date of filing: 15.02.2016
(51) Int. Cl.: G06F 19/00

(54) **CHRONOLOGICAL CHANGE PREDICTION SYSTEM**

(30) Priority: 18.02.2015 JP 2015029911
(71) Applicant: Hitachi, Ltd., Tokyo (JP)
(72) Inventor: HIROKI, Keiichi, Chiyoda-ku, Tokyo 100-8280 (JP); MIYOSHI, Toshinori, Chiyoda-ku, Tokyo 100-8280 (JP)
(74) Representative: Moore, Graeme Patrick

(57) **Abstract**

A chronological change prediction system stores a graphical model which an evidence set at a first time is inputted in and outputs information about states of random variables at a second time when a first period has passed from the first time, determines whether to convert each probability distribution in the first evidence set based on a feature value calculated from the probability distributions and/or a feature value calculated from a random variable associated with the probability distributions in the graphical model, converts each probability distribution determined to be converted into a particular state of a random variable corresponding to each probability distribution determined to be converted, creates a second evidence set from the first evidence set by replacing each probability distribution determined to be converted and included in the first evidence set with the first particular state, and inputs the second evidence set to the graphical model.

## Description

### CLAIM OF PRIORITY

The present application claims priority from Japanese patent application JP 2015-29911 filed on February 18, 2015, the content of which is hereby incorporated by reference into this application.

### BACKGROUND

The present invention relates to a chronological change prediction system.

Background arts for supporting health business include JP 2014-225176. JP 2014-225176 discloses that "a system comprising: a causation/transition structure calculating unit generating a graph structure including a node and a probability variable relating to item, and a probabilistic dependency defined by one of a directed link or an undirected link between the nodes; a node generating unit generating an event space of the nodes; a probability calculating unit calculating a conditional probability of the graph structure; a state transition model reconstructing unit reconstructing a state transition model with a graph structure, an event space and a conditional probability including specified probability variables based on a state transition model; a disease state transition estimating unit estimating a disease state transition probability based on the reconstructed state transition model; and a health guidance supporting unit selecting a subject for health guidance and a content of health guidance based on the estimated disease state transition probability."

### SUMMARY

JP 2014-225176 constructs, with data about states of a certain year and the next year, a Bayesian network which predicts a one-year-later prevalence rate and one-year-later medical expenses. Evidence which is inputted to a graphical network, such as the Bayesian network, includes hard evidence, which is information about a particular state of a random variable, and soft evidence, which is information about a probability distribution in the case that the probability distribution of a random variable which still has a plurality of states is proved.

The art of JP 2014-225176 predicts a one-year-later state by inputting the hard evidence which is information about present states to the Bayesian network. Thus, when the present state includes a probability distribution of a random variable (which still has a plurality of states), the art of JP 2014-225176 must converts the probability distribution into a particular state.

Methods for converting a probability distribution into a particular state include a method which selects a mode, which is a state that appears most often in the probability distribution, as the particular state and a method which calculates an expectation value with the probability distribution and selects a state corresponding to the expectation value as the particular state. When the particular state converted from the probability distribution is inputted, prediction accuracy decreases as compared to a case that the probability distribution is inputted.

When prediction which inputs a result of the last prediction by the graphical model into the graphical is repeated, a result of the repeated prediction should greatly differ from a future state. The prevalence rates and the medical expenses are likely to increase with ages. However, chronological change prediction with particular states into which the prevalence rates and the medical expenses are converted should create prediction results that the prevalence rates and the medical expenses decrease year by year.

Deviations of probability distributions of them cause the problem. The number of people who are suffered with a certain disease is typically much smaller than the number of people who are suffered with the disease. Thus, a probability distribution about whether the disease has emerged or not would be converted into a state of the disease having emerged. As a result, the repeated prediction make states of almost all the people converge to the states of the people being suffered with the disease.

In addition, when the soft evidence is inputted to the graphical model, a calculation amount of the prediction increases with, for example, the number of states of the random variables. An aspect of the present invention provides a chronological change prediction system. By properly selecting, from probability distributions included in an evidence set, a probability distribution to be converted into a particular state of a random variable corresponding to the random probability, the chronological change prediction system enables prediction with higher accuracy and a lower calculation amount.

The aspect of the present invention adopts, for example, the following configuration for solving the above problems. A chronological change prediction system predicting states of random variables with a graphical model, the chronological change prediction system comprising: a processor; and a memory device, wherein the memory device is configured to store the graphical model, wherein an input of the graphical model is a first evidence set at a first time, wherein an output of the graphical model is information about states of the random variables at a second time when a first period has passed from the first time, wherein the first evidence set includes probability distributions of all or a part of the random variables, wherein the processor is configured to execute prediction processing with the graphical model, and wherein, in the prediction processing, the processor is configured to: obtain the first evidence set; determine whether to convert each of the probability distributions based on a feature value calculated from each of the probability distributions and/or a feature value calculated from a random variable associated in the graphical model with each of the probability distributions; convert each probability distribution determined to be converted into a first particular state of a first random variable corresponding to each probability distribution determined to be converted; create a second evidence set from the first evidence set by replacing each probability distribution determined to be converted and included in the first evidence set with the first particular state; input the second evidence set to the graphical model; and output information about states of the random variables of the second time.

The aspect of the present invention enables prediction with high accuracy and a low calculation amount by using a graphical model predicting a state of random variable in a certain period later.

Problems, configurations, and effects which are not mentioned above are explained in the following embodiments.

### BRIEF DESCRIPTIONS OF DRAWINGS

The present invention can be appreciated by the description which follows in conjunction with the following figures, wherein:
FIG. 1 is a block diagram depicting an example of a configuration of a disease onset prediction apparatus;
FIG. 2A is an example of a configuration of a random variable table;
FIG. 2B is an example of a configuration of a link table;
FIG. 2C is an example of a configuration of a probability table;
FIG. 3C is an example of a configuration of a probability distribution table for output random variables;
FIG. 4 is an example of a configuration of an S/H select table;
FIG. 5 is an example of a configuration of a mixed evidence table;
FIG. 6 is a flowchart depicting an example of disease onset prediction processing;
FIG. 7 is a flowchart depicting an example of S/H select processing;
FIG. 8 is an example of an interface for an S/H condition set unit setting a condition;
FIG. 9A is an example of a Bayesian network consisting of sets including random variables depending on each other;
FIG. 9B is an example of patterns having blocked paths;
FIG. 9C is an example of patterns not having blocked paths;
FIG. 10A is an example of input and output for predicting future disease onset and medical expenses;
FIG. 10B is an example of input and output for predicting future measured values based on life style;
FIG. 10C is an example of input and output for inferring life style;
FIG. 11A is an example of an interface showing output of the disease onset prediction apparatus; and
FIG. 11B is an example of another interface showing output of the disease onset prediction apparatus.

### DETAILED DESCRIPTION OF EMBODIMENTS

In the following, an embodiment of the present invention is explained referring the attached drawings. The embodiment is an example to achieve the present invention and does not limit a technical range of the present invention. In the drawings, the same configuration has the same reference letter.

By using soft evidence inference whose input includes probability distributions of random variables, a chronological change prediction system in the embodiment achieves chronological change prediction with a prediction model which predicts a one-year-later state. When inputting a probability distribution to the prediction model, the chronological change prediction system need not convert a probability distribution of the random variables into a particular state of the random variable. Thus the chronological change prediction system enables prediction with high accuracy. In addition, when repeating prediction which inputs the outputted probability distribution to the prediction model, the chronological change prediction system prevents values of the random variables from converging to incorrect values.

On the other hand, the stochastic inference processing with the soft evidence has a problem that a calculation amount increases with, for example, the number of states of the random variables to which the soft evidence inputted. Thus, when the chronological change prediction system inputs the soft evidence to, for example, all input random variables, the calculation amount of the prediction increases.

Based on a feature value of each of the input random variables and/ or a feature value of probability distribution of an output random variable corresponding to each of the input random variables, the chronological change prediction system in the embodiment selects whether the soft evidence or the hard evidence is inputted to each of the input random variables. In addition, the chronological change prediction system may determine a method for converting the soft evidence into the hard evidence by using, for example, a feature value of each of the input random variables. The above processing enables the chronological change prediction system to execute prediction with higher accuracy and a smaller calculate amount.

### <Embodiment 1 >

This embodiment explains an example of a disease onset prediction apparatus, which is an example of the chronological change prediction system. The disease onset prediction apparatus predicts chronological change of analysis subjects for each predetermined period based on medical data, such as medical examination results, medical interview results, medical histories, and medical records. The predetermined period in this embodiment is one year.

The medical data includes information about medical care and health for each person, such as medical reports and medical test values. The medical data may include values measured in medical examinations or medical interviews, such as heights, weights, BMIs, blood pressures, cholesterol levels, and blood-sugar levels. The medical data may include data on life style, such as smoking, exercise, drinking, and sleep conditions. The medical data may include medical histories, or histories of injury and disease in seeing medical institutions. The medical data may include data on medical records including prescribed medicines, medical practices, and medical expenses.

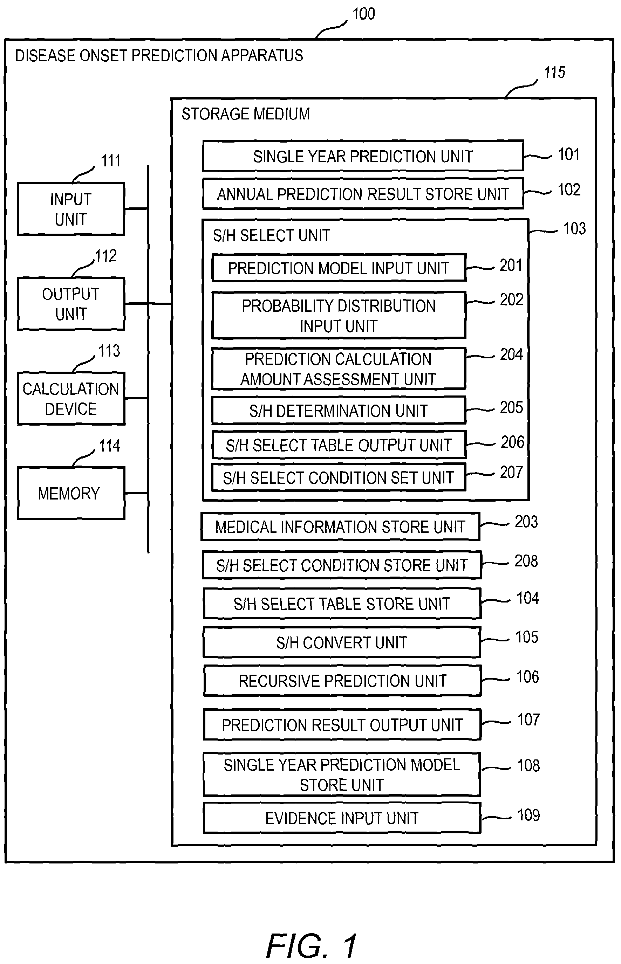

FIG. 1 depicts an example of the configuration of the disease onset prediction apparatus. The disease onset prediction apparatus 100 includes an input unit 111, an output unit 112, a calculation device 113, a memory 114, and a storage medium 115. The input unit 111, which is, for example, a human interface, such as a mouse or a keyboard, receives data and the like from users. The output unit 112, which is, for example, a display or a printer, outputs results calculated by the disease onset prediction apparatus 100. The storage medium 115 stores programs achieving processing by the disease onset prediction apparatus 100 and data, such as the calculation result.

The calculation device 113 is, for example, a CPU or a GPU. The calculation device 113 includes, for example, a processor and/or a logical circuit which operates according to the programs, inputs/outputs data, reads/writes data, and runs the programs. The calculation device 113 executes processing and calculation which are explained hereinafter.

The memory 114 loads data and the programs run by the calculation device 113 and stores them temporarily. The memory 114 may store all or a part of data stored in the storage medium 115. The programs may be stored in a computer-readable and transportable non-transitory storage medium and be loaded into the memory 114 through an external storage device reading the non-transitory storage medium as necessary.

The storage medium 115 includes a single year prediction unit 101, an S/H select unit 103, an S/H convert unit 105, a recursive prediction unit 106, a prediction result output unit 107 and an evidence input unit 109, which are programs. The storage medium 115 includes an annual prediction result store unit 102, a medical information store unit 203, an S/H select condition store unit 208, an S/H select table store unit 104, and a single year prediction model store unit 108, which are areas storing data.

The programs, which are run by the calculation device 113, execute processing described therein with a communication port (a communication device). In this embodiment, a sentence whose subject word is a program may be replaced with a sentence whose subject word is the calculation device 113. Processing executed by a program is processing executed by a computer and a computer system which run the program.

The calculation device 113 operates as function units (means) achieving predetermined functions by operating according to the programs. For example, the calculation device 113 operates as a single year prediction unit (a single year prediction mean) by operating according to the single year prediction unit 101 and as an S/H select unit (an S/H select mean) by operating according to the S/H select unit. That can also be said about the other programs. In addition, the calculation device 113 operates as function units (means) achieving each piece of the processing executed by each of the programs. The computer and the computer system are a device and a system which include the function units (means), respectively.

The single year prediction model store unit 108 stores, for example, information about a Bayesian network inputted by, for example, users in advance. The Bayesian network receives medical data indicating a state of a certain point in time and predicts a state of a point when one year has passed from the certain point by using the medical data. The Bayesian network hereinafter also referred to as a single year prediction model. The single year prediction model store unit 108 stores a random variable table, a link table, and a probability table which are mentioned below. The single year prediction model is constructed with statistical methods based on past medical data and describes statistical causal association between items in the past medical data.

The causal association between the items includes, for example, association between disease and prescribed medicines for the disease, association between abnormal values in medical tests and symptoms indicated by the abnormal values, and association between disease and increase of medical expenses caused by the disease.

The single year prediction model includes at least two types of random variables. The at least two types of random variables include input random variables indicating a state of a certain point in time and output random variables indicating a state of the point when one year has passed from the certain point. When including an input random variable indicating whether disease A has emerged at a certain point in time, the single year prediction model includes an output random variable indicating whether disease A will have emerged at the point when one year has passed from the certain point.

The single year prediction unit 101 executes prediction processing receiving medical data indicating a state of a certain point in time as an input and outputting a state of the point when one year has passed from the certain point. Each piece of the input, such as the medical data, is called evidence. The evidence is inputted in the input random variables.

The evidence includes the two types of evidence, which are hard evidence and soft evidence. The hard evidence is information about a particular state in the case that a random variable comes to have the particular state. Information about a state of "disease A having emerged" is an example of the hard evidence. Information about a state of a test value being in a particular range of predetermined ranges, such as information about a state of "a blood-sugar level being in a range from 80 mg/dl to 100 mg/dl," is also an example of the hard evidence.

The soft evidence is information about a probability distribution in the case that the probability distribution of a random variable which still has a plurality of states is proved. Information about a state of "a probability that disease A has emerged being 15%" and information about a state of "a probability that a blood-sugar level is less than or equals to 80 mg/dl being 10%, a probability that the blood-sugar level is in a range from 80 mg/dl exclusive to 100 mg/dl inclusive being 50%, and a probability that the blood-sugar-level is greater than 100 mg/dl being 40%" are examples of the soft evidence.

The annual prediction result store unit 102 stores a prediction result outputted by the single year prediction unit 101, or a probability distribution table indicating a probability distribution for each of the output random variables. The configuration of the probability distribution table is to be described below. When the disease onset prediction apparatus 100 predicts chronological change, the annual prediction result store unit 102 stores a prediction result of each year.

The S/H select unit 103 selects which type of the evidence is inputted to each of the input random variables, the soft evidence or the hard evidence. The S/H select unit 103 creates an S/H select table indicating the selection result. The configuration of the S/H select table is described below. In addition, when selecting the hard evidence for a random variable, the S/H select unit 103 may determine a method for converting the soft evidence or a probability distribution of the random variable into the hard evidence.

The S/H select unit 103 includes a prediction model input unit 201, a probability distribution input unit 202, a prediction calculation amount assessment unit 204, an S/H determination unit 205, an S/H select table output unit 206, and an S/H select condition set unit 207.

The prediction model input unit 201 obtains a single year prediction model as an input from the single year prediction model store unit 108. The probability distribution input unit 202 obtains probability distributions of input random variables as an input from the annual prediction result store unit 102. The prediction calculation amount assessment unit 204 assesses, with the single year prediction model and the S/H select table, a calculation amount required for a single year prediction. The S/H determination unit 205 determines which type of the evidence is inputted to each of the input random variables in the single year prediction model, the soft evidence or the hard evidence. The S/H determination unit 205 and creates the S/H select table. The S/H select table output unit 206 outputs the created S/H select table to the output unit 112. The S/H select condition set unit 207 receives a condition, through the input unit 111 from users and the like, for the S/H determination unit 205 determining.

The medical information store unit 203 stores information about association between the random variables in the single year prediction model. The information may be inputted by users in advance. The information may include information which differs from association information included in the single year prediction model. Correlation coefficients of random variables are an example of the association. Existence or non-existence of association between random variables is also an example of the association in the medical information store unit 203. The medical information store unit 203 may store information about association between input random variables and information about association between output random variables.

The S/H select condition store unit 208 stores the select condition that the S/H select condition set unit 207 receives. The S/H select table store unit 104 stores the S/H select table created by the S/H select unit 103.

The S/H convert unit 105 converts all or a part of items of a prediction result stored in the annual prediction result store unit 102 into the hard evidence according to the S/H select table stored in the S/H select table store unit 104. Items which are not converted into the hard evidence remain probability distributions and are treated as the soft evidence. The hard evidence which includes the converted items and the soft evidence are used as an input for next prediction processing by the single year prediction unit 101.

The recursive prediction unit 106 executes recursive chronological change prediction with, for example, the single year prediction unit 101, the annual prediction result store unit 102, the S/H select unit 103, the S/H select table store unit 104, the S/H convert unit 105, the prediction result output unit 107, and the single year prediction model store unit 108. The prediction result output unit 107 outputs a prediction result of each year stored in the annual prediction result store unit 102 to the output unit 112. The evidence input unit 109 receives evidence as an input from users through the input unit 111. The inputted evidence is used for the first prediction by the single year prediction unit 101.

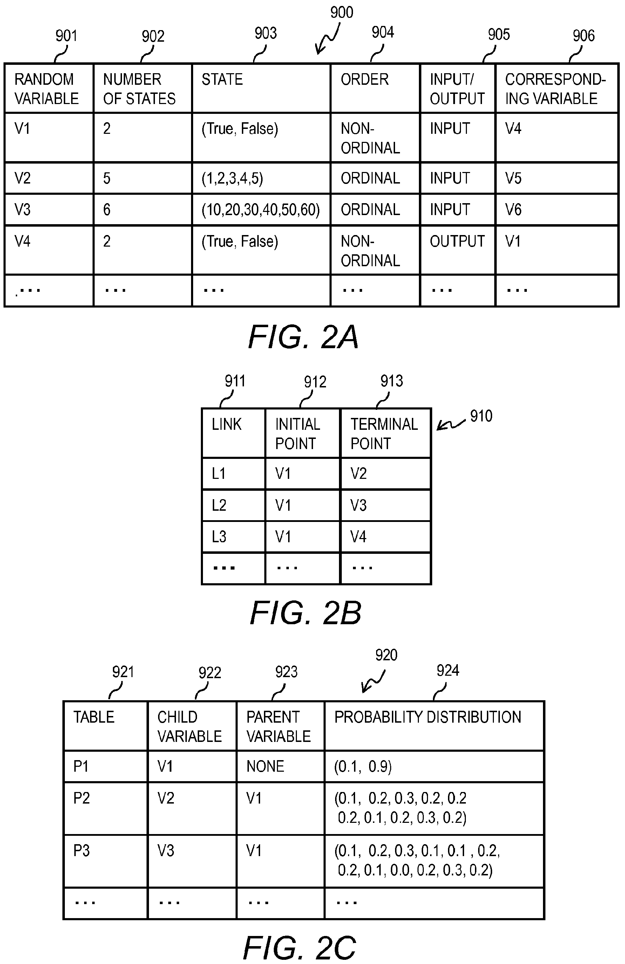

The configurations of tables stored in the disease onset prediction apparatus 100 are explained below. FIG. 2A, FIG. 2B, and FIG. 2C depict the configuration of the single year prediction model, or the Bayesian network. The Bayesian network is identified by the random variable table 900, the link table 910, and the probability table 920 which are stored in the single year prediction model store unit 108.

FIG. 2A depicts an example of the configuration of the random variable table 900. The random variable table 900 indicates characteristics of random variables, which are nodes included in the Bayesian network. The random variable table 900 includes, for example, a random variable column 901, a number of states column 902, a state column 903, an order column 904, an input/output column 905, and a corresponding variable column 906.

The random variable column 901 stores the random variables which are nodes included in the Bayesian network. The number of states column 902 stores the number of possible states of each of the random variables stored in the random variable column 901. The state column 903 stores the possible states of each of the random variables. The order column 904 indicates whether each of the random variables is an ordinal random variable or not, that is, whether a set consisting of the states of the random variable is an ordered set or not.

The input/output column 905 indicates that each of the random variables is an input random variable or an output random variable. The corresponding variable column 906 stores output random variables corresponding to input random variables stored in the random variable column 901 and stores input random variables corresponding to output random variables stored in the random variable column 901. When there are no input random variables and no output random variables which correspond to a random variable stored in the random variable column 901, the corresponding variable column 906 stores, for example, a null value or the value "none."

FIG. 2B depicts an example of the configuration of the link table 910. The link table indicates causal association between the random variables included in the Bayesian network. The link table 910 includes a link column 911, an initial point column 912, and a terminal point column 913. The link column 911 stores link names and indicates that random variables stored in the initial point column 912 are causally associated with corresponding random variables stored in the terminal point column 913. In the association between two random variables, random variables which are initial points are referred to as parent variables, and random variables which are terminal points are referred to as child variables.

FIG. 2C depicts an example of the configuration of the probability table 920. The probability table 920 indicates probability distributions of random variables included in the Bayesian network, and the probability distributions depend on states of parent variables of the random variables. The probability table 920 includes, for example, a table column 921, a child variable column, a parent variable column, and a probability distribution column 924. The table column 921 stores identifiers of records of the probability table 920. The child variable column stores child variables. The parent variable column 923 stores parent variables.

The probability distribution column 924 stores matrixes. Each row of the matrixes indicates a probability distribution of a child variable, according to a state of a parent variable corresponding to the child variable, stored in the child variable column 922 stores. Cells of the probability distribution column 924 corresponding to the cells "none" of the parent variable column 923 store prior probability distributions of the child variables. The number of parent variables corresponding to a child variable may be zero and be greater than one.

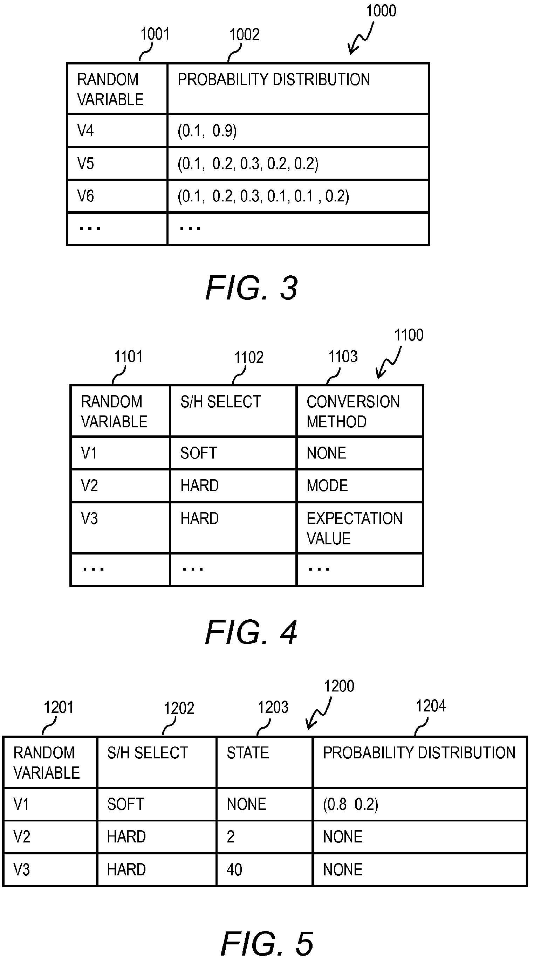

FIG. 3 depicts an example of the configuration of the probability distribution table for the output random variables which is outputted by the prediction result output unit 107. The probability distribution table 1000 includes, for example, a random variable column 1001 and a probability distribution column 1002. The random variable column 1001 stores the output random variables. The probability distribution column 1002 stores probability distributions of the output random variables.

FIG. 4 depicts an example of the configuration of the S/H select table. The S/H select table 1100 includes, for example, a random variable column 1101, an S/H select column 1102, and a conversion method column 1103. The random variable column 1101 stores the input random variables. The S/H select column 1102 indicates which type of the evidence is inputted into each of the input random variables, the soft evidence or the hard evidence. In the S/H select column 1102, "soft" and "hard" mean the soft evidence and the hard evidence, respectively. Cells of the conversion method column 1103 corresponding to the cells "hard" of the S/H select column store methods for converting probability distributions into the hard evidence.

FIG. 5 depicts an example of the configuration of the mixed evidence table. The mixed evidence table includes, for example, a random variable column 1201, an S/H select column 1202, a state column 1203, and a probability distribution column 1204. The random variable column 1201 stores random variables. The S/H select column 1202 indicates which type of the evidence is inputted into each of the input random variables, the soft evidence or the hard evidence.

Each cell of the state column 1203 corresponding to each of the cells "hard" of the S/H select column 1202 stores a particular state of the input random variable of the record. Cells of the state column 1203 corresponding to cells of "soft" of the S/H select column 1202 store probability distributions of the input random variables. Cells of the probability distribution column 1204 corresponding to cells of "hard" of the S/H select column 1202 store "none."

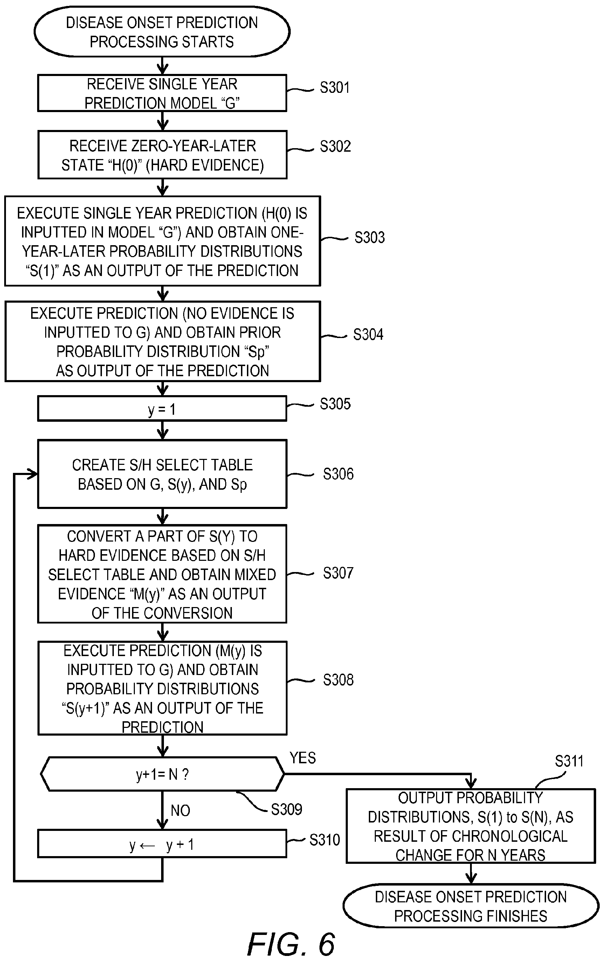

An example of operation of the disease onset prediction apparatus 100 is explained below. FIG. 6 depicts an example that the disease onset prediction apparatus 100 predicts N-year chronological change of a disease onset probability. The disease onset prediction apparatus 100 executes N-year-later prediction by using the single year prediction model repeatedly. By properly selecting the soft evidence or the hard evidence based on feature values of random variables in the single year prediction model, the disease onset prediction apparatus 100 improves prediction accuracy and regulates the amount of calculation. Furthermore, the disease onset prediction apparatus 100 prevents results by repeated prediction from converging to incorrect values.

In a step S301, the prediction model input unit 201 receives information about the single year prediction model, or information on the random variable table 900, the link table 910, and the probability table 920 as an input. In a step S302, the evidence input unit 109 receives a set H(0), which consists of particular zero-year-later states of at least one random variable for prediction targets and is the initial input for the whole prediction. It is assumed that all elements of the set H(0) is the hard evidence.

In a step S303, the single year prediction unit 101 executes first prediction with the single year prediction model. Input of the first prediction is the H(0), and output of the first prediction is an S(1), which consists of at least one probability distribution indicating a one-year-later prediction result. The single year prediction unit 101 stores the probability distributions included in the S(1) in the probability distribution table 1000. In a step S304, the single year prediction unit 101 obtains a set Sp, which consists of at least one prior probability distribution, by executing prediction with the single year prediction model without input.

In a step S305, the recursive prediction unit 106 sets y (where y is passed years) to 1 and starts recursive prediction. In a step S306, the S/H select unit 103 creates the S/H select table 1100 with the single year prediction model, the S(y), and the Sp. Processing which creates the S/H select table 1100 is mentioned below.

In a step S307, the S/H convert unit 105 converts an S(y), which is a y-year-later prediction result, into an M(y), which is y-year-later mixed evidence. The mixed evidence M(y) is a set including probability distributions indicated by the soft evidence and particular states indicated by the hard evidence. Each of the probability distributions and the particular states is input to the input random variables. The mixed evidence table 1200 stores information about the mixed evidence M(y).

Specifically, in the step 307, the S/H convert unit 105 obtains values of the random variable column 1101 and the S/H select column 1102 in the S/H select table 1100 and stores obtained values in the random variable column 1201 and the S/H select column 1202 in the mixed evidence table 1200.

As for input random variables whose values of the S/H select column 1202 are "soft," the S/H convert unit 105 stores "none" and probability distributions of the input random variables of the S(y) in the state column 1203 and the probability distribution column 1204, respectively.

The S/H convert unit 105 converts each probability distribution of input random variable of the S (y) whose values of the S/H select column 1202 are "hard" into a particular state with a method stored in the conversion method column 1103. As for the input random variables whose values of the S/H select column 1202 are "hard," the S/H convert unit 105 stores the particular states and "none" in the state column 1203 and the probability distribution column 1204 in the mixed evidence table 1200, respectively.

In a step S308, the single year prediction unit 101 receives the mixed evidence M(y) as an input and executes y-year-later prediction with the mixed evidence M(y). In a step 309, the recursive prediction unit 106 determines whether "y+1=N" is fulfilled. When determining that "y+1<N" is fulfilled (S309: NO), the recursive prediction unit 106 proceeds to a step S310. In the step S310, the recursive prediction unit 106 adds 1 to the y, and then recedes to the step S306.

In the step 309, when determining that "y+1=N" is fulfilled, that means a N-year-later prediction result is obtained, the recursive prediction unit 106 proceeds to a step S311. In the step S311, the prediction result output unit 107 outputs the S(1), an S(2), ..., and an S(N), which are the prediction results, and then the disease onset prediction apparatus 100 finishes the disease onset prediction. Though it is assumed that all elements in the H(0) are the hard evidence in the step S302, the H(0) may include probability distributions of the random variables, or the soft evidence. In this case, the S/H select unit 103 may determine that the S/H convert unit 105 converts the each of the probability distributions of the soft evidence, and the S/H convert unit 105 may execute the converting according to the determination.

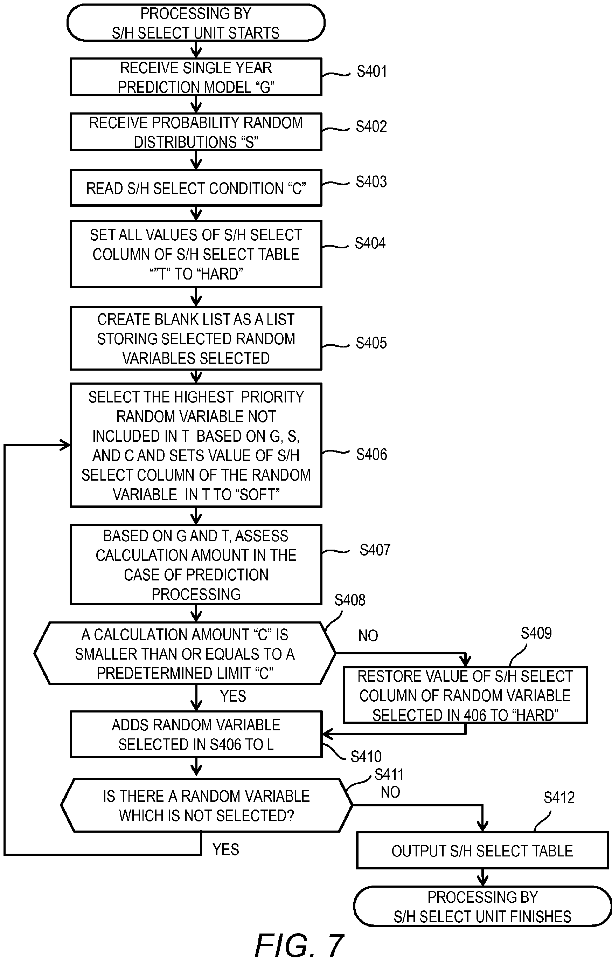

An example of operation of the S/H select unit 103 is explained below. FIG. 7 depicts an example of operation of the S/H select unit 103. In a step S401, the prediction model input unit 201 receives information on the single year prediction model, or information on the random variable table 900, the link table 910, and the probability table 920 from the single year prediction model store unit 108. In a step S402, the probability distribution input unit 202 obtains probability distributions included in the S(y), which is information on the probability distribution table 1000, from the annual prediction result store unit 102.

In a step S403, the S/H determination unit 205 reads in S/H select conditions stored in the S/H select condition store unit 208. The S/H select conditions, which are used in creating the S/H select table 1100, may be predetermined by users. The S/H select conditions include rules for determining priorities of random variables for inputting the soft evidence without converting into the hard evidence. The rules are based on, for example, a calculation amount upper limit for one-time prediction and output random variables which should be predicted accurately.

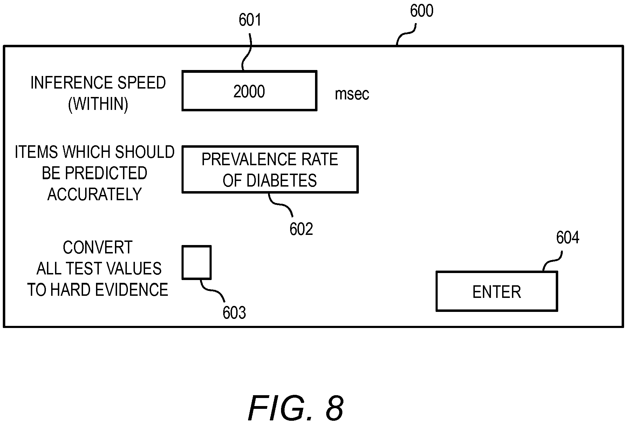

FIG. 8 depicts an example of a user interface for the S/H select condition set unit 207 for setting the S/H select conditions. The user interface 600 for setting includes, for example, input areas 601 to 602, a check box 603, and an enter button 604. The input area 601 receives input of an inference speed. The inference speed may be an inference speed of a loop from the steps S306 to S310 or an inference speed of the steps S301 to S311.

The input area 602 receives input of output random variables which should be predicted accurately. The check box 603 is a check box for converting all probability distributions corresponding to test values into the hard evidence. The enter button 604 is a button for entering the S/H select condition inputted in the input areas 601 to 602 and the check box 603.

The explanation of FIG. 7 returns. In a step S404, the S/H determination unit 205 stores each of the input random variables stored in the random variable table 900 in the random variable column 1101 of the S/H select table 1100 and, for example, sets all values of the S/H select column 1102 to "hard." The S/H determination unit 205 identifies probability distributions of output random variables corresponding to the input random variables by referring the random variable table 900.

The S/H determination unit 205 determines converting methods, by referring the random variable table 900, according to features of the random variables stored in the random variable column 1101. For example, the S/H determination unit 205 sets values of the conversion method column 1103 which correspond to ordinal random variables to "expectation value." For example, the S/H convert unit 105 converts a probability distribution of a random variable whose value of the conversion method column 1103 is "expectation value" into ,for example, the state which is the nearest to an expectation value of the probability distribution.

As for each pair of neighboring values included in an ordinal random variable, the S/H determination unit 205 may calculate differences between a greater value and a smaller value of the pair. When determining that a variance of the calculated differences is greater than or equals to a predetermined value, the S/H determination unit 205 may set values of the conversion method column 1103 which correspond to the ordinal random variable to "mode."

For example, the S/H determination unit 205 may set values of the conversion method column 1103 which correspond to non-ordinal random variables to "mode". The S/H determination unit 205 improves prediction accuracy by the above mentioned determination of the converting methods. The random variable table 900 may store methods for converting the input random variables into the hard evidence in advance, and the S/H determination unit 205 may store the methods in the conversion method column 1103.

In a step S405, the S/H determination unit 205 creates, for example, a blank list as a list storing random variables selected in a step S406. In the step S406, the S/H determination unit 205 selects an input random variable having the highest priority from random variables which are not included in the selected random variables list. The S/H determination unit 205 selects the highest priority input random variable with, for example, the single year prediction model, the probability distributions included in the S(y), and the S/H select condition. The S/H determination unit 205 sets a value of the S/H select column 1102 of the highest priority random variable to "soft." Probability distributions of random variables whose values of the S/H select column 1102 are "hard" at this time are candidates for being converted into the hard evidence. When there are two input random variables having the highest priority, the S/H determination unit 205 may select one random variable from the two random variables at random.

The S/H determination unit 205 determines a priority of an input random variable with feature values of the input random variable, such as a deviation of the prior probability distribution of the input random variable, the number of states of the input random variable, and a value indicating whether the input random variable is ordinal. The S/H determination unit 205 may determine the priority with feature values of the probability distribution of the input random variable, such as an entropy value of the input random variable. For example, the smaller the number of states of a random variable is, the higher a priority of the random variable the S/H determination unit 205 determines. In this manner, the S/H determination unit 205 can select input random variables which hardly increase the calculation amount.

For example, the greater an entropy value of a random variable and/or a deviation of a prior probability distribution of the random variable is, the higher a priority of the random variable the S/H determination unit 205 determines. Further, the S/H determination unit 205 may give a higher priority to an ordinal random variable than a non-ordinal random variable. In this manner, the S/H determination unit 205 can select input random variables which greatly contribute to prediction accuracy

The S/H determination unit 205, for example, obtains the feature values, such as the numbers of the input random variables and the value indicating whether the input random variable is ordinal or not, from the random variable table 900. The S/H determination unit 205, for example, calculates the deviations of the prior probability distributions of the input random variables using probability distributions obtained from the probability table 920. The S/H determination unit 205 may obtain probability distributions of output random variables from the probability distribution table 1000, calculate entropy values of the probability distributions, and determines the calculated entropy values as entropy values of input random variables corresponding to the output random variables.

In the case where there are a random variable X, which indicates whether a medicine A is dosed, and a random variable Y, which indicates values of test B, the S/H determination unit 205 determines the priority of the random variable X higher. Since the random variable X includes only two states, which are "dosed" and "not dosed", and since a probability of "dosed" is very small, an entropy value of the random variable X is great.

On the other hand, since the random variable Y includes many states, which are numerical values, and since a deviation of a probability distribution of the random variable Y is not so great, an entropy value of the random variable Y is small. Since the random variable Y is ordinal, an expectation value of the random variable Y can be calculated. Thus, the S/H determination unit 205 enables the calculation amount to reduce and prevents the prediction accuracy from descending by determining that a priority of the random variable X is higher than that of the random variable Y.

The S/H determination unit 205 may determine priorities of random variables by using a plurality of types of feature values of the random variables. In this case, the S/H determination unit 205 may calculate a value for each of the random variables by substituting the feature values of the random variable for a predetermined function. The S/H determination unit 205 may give high priorities to the random variables in descending order of the calculated values of the random variables. For example, the smaller the number of states and/or the value indicating whether the random variable is ordinal or not (for example, the value is 1 if the random variable is ordinal, and otherwise the value is 0) and/or the greater a value of entropy is, the greater a value of the random variable calculated from the predetermined function is.

In the case of the plurality of types of the feature values, the S/H determination unit 205 may determine the priorities by sorting the input random variables by the types of the feature values with predetermined sorting priorities of the types of feature values. When the types of the feature values consist of the number of states having a high sorting priority, the entropy value having a middle sorting priority, and the value having a low sorting priority and indicating whether a random variable is ordinal or not, the S/H determination unit 205 may determine priorities of the random variables by sorting the input random variables ascending order by a first key, which is the number of states, in descending order by a second key, which is the entropy value, and in ascending order by a third key, which is the value indicating whether a random variable is ordinal or not. The S/H determination unit 205 may determine the priorities of the random variables in accordance with the ranking of the sorted input random variables.

When the S/H select condition includes items which should be predicted accurately, the S/H determination unit 205 may raise priorities of random variables corresponding to the items and/or priorities of random variables corresponding to items which are associated, by information stored in the medical information store unit 203, with the items which should be predicted accurately. Specifically, the S/H determination unit 205 may raise the priorities of the determined random variables to the highest priority. In the case where the predetermined function is used for determining the priorities, as for the determined random variables, the S/H determination unit 205 may add predetermined values to the values calculated from the predetermined function. The S/H determination unit 205 can use association which differs from the association indicated by the Bayesian network by using information stored in the medical information store unit 203 and result in higher prediction accuracy.

In a step S407, the prediction calculation amount assessment unit 204 assesses a prediction calculation amount with the single year prediction model and the S/H select table 1100 of the year. In a step 408, the prediction calculation amount assessment unit 204 determines whether the assessed calculation amount is less than or equals to a predetermined upper limit value included in the S/H select condition.

When determining that the assessed calculation amount is greater than the upper limit value (S408: NO), the prediction calculation amount assessment unit 204 proceeds to a step S409. In the step S409, the S/H determination unit 205 restores the value of the S/H select column 1102 of the input random variable selected in the step S406 to "hard" and proceeds to a step S410. When determining that the assessed calculation amount is less than or equals to the upper limit value (S408: YES), the prediction calculation amount assessment unit 204 proceeds to a step S411.

In the step S410, S/H determination unit 205 includes the input random variable selected in the step S406 in the selected random variables list. In a step S411, the S/H determination unit 205 determines whether there exists an input random variable which is included in the S/H select table 1100 and is not selected in the step S406.

When determining that there exist the input random variable (S411: YES), the S/H determination unit 205 recedes to the step S406. When the S/H determination unit 205 determines that there exist no input random variable which is included in the S/H select table 1100 and is not selected in the step S406 (S411: NO), the S/H select table output unit 206 outputs the S/H select table 1100 to the S/H select table store unit 104 in a step S413. Then the processing by the S/H select unit 103 finishes.

The S/H select unit 103 does not have to create the S/H select tables 1100 for every prediction year. For example, the S/H select unit 103 creates S/H select tables of each year from one year later to M years later inclusive (where M, which is less than N, is a natural number) and executes the M+1-year-later S/H conversion with the M-year-later S/H select table 1100.

There are feature values which are constant with respect to years, such as the number of states, the deviation of a probability distribution, and the value indicating whether a random variable is ordinal or not. Thus, when creating the S/H select table 1100 with at least one of the feature values, the S/H determination unit 205 may create only a one-year-later S/H select table 1100. The S/H select unit 103 may change methods for determining values of the S/H select column 1102 and the conversion method column 1103 for each prediction year.

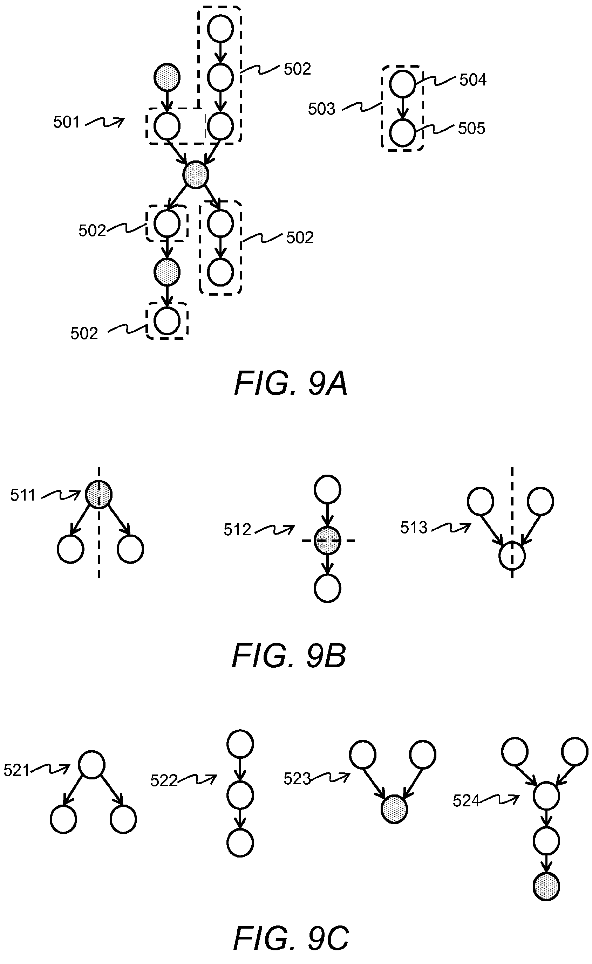

In the following, an example of processing of the step S408 which assesses the prediction calculation amount is explained. FIG. 9A depicts an example of a Bayesian network consisting of sets including random variables depending on each other. In FIG. 9A and after-mentioned FIG.9B and FIG. 9C, each circle is a node, and each arrow is causal association between the nodes, and the inside of each of the circles indicates a type of evidence inputted into the node. In FIG. 9A, FIG.9B, and FIG. 9C, each circle not filled with dots is a first type node, and each circle filled with dots is a second type node. The first type node is a node in which the soft evidence is inputted in or a node in which no evidence is inputted. The second type node is a node in which the hard evidence inputted in.

The construction of a Bayesian network 501 is identified by the link table 910, and a type of evidence which is inputted in each node of the Bayesian network 501 is identified by the S/H select table 1100 of the initial time. With the types of nodes and paths connecting nodes, the prediction calculation amount assessment unit 204 obtains random variables, which are nodes receiving inputs except for the hard evidence, from the Bayesian network 501 and identifies each random variable set 502 by classifying the obtained random variables into dependent random variable groups. A method for identifying the random variable sets 502 is explained below.

As for each of the random variable sets 502, the prediction calculation amount assessment unit 204 may obtain random variables in which the soft evidence is inputted from the random variable set 502, calculate a product of states of the obtained random variables, and determine a calculation amount of the random variable set 502 as the product. The prediction calculation amount assessment unit 204 may calculate a sum of the calculation amounts of all the random variable sets 502 and determine a calculation amount for inferring states of all the random variables, which is the calculation amount of the step S408, as the sum.

In the following, the method for identifying the random variable sets 502 is explained. A Bayesian network 503 includes a random variable 504 and a random variable 505 which are linked each other. Since the Bayesian network 503 includes only one link, the random variable 504 and the random variable 505 do not depend on all other random variables. Thus, the prediction calculation amount assessment unit 204 determines that the Bayesian network consists of a random variable set consisting of the random variable 504 and the random variable 505.

The prediction calculation amount assessment unit 204 repeats processing which determines a set consisting of random variables included in a path as a random variable set. Even if there is a path between random variables, the path can be blocked depending on the existence of a node in which the hard evidence is inputted and the structure of the graph. When there is a path between two random variables, the two random variables do not depend on each other.

FIG. 9B depicts patterns having blocked paths. Patterns 511 to 513 include paths blocked by dashed lines. The pattern 511, which is called a tail-to-tail pattern, includes paths from a parent node to child nodes. The parent node in the pattern 511 is the second type node. The child nodes in the pattern 511 are the first type nodes.

The pattern 512, which is called a head-to-tail pattern, includes a path from a parent node to a child node and a path from the child node to a grandchild node. The parent node is in the pattern 512 is the first type node. The child node in the pattern 512 is the second type node. The grandchild node in the pattern 511 is the first type node. The pattern 513, which is called a head-to-head pattern, includes paths from parent nodes to a child node. The parent nodes and the child node in the pattern 513 are the first type nodes.

FIG. 9C depicts patterns not having blocked paths. A parent node and childe nodes in a pattern 521, which is tail-to-head, are the first type nodes. A parent node, a child node, and a grandchild node in a pattern 522, which is head-to-head, are the second type nodes. A pattern 523 and a pattern 524, which are head-to-head, include parent nodes, which are the first type nodes, and descendent nodes which are the second type nodes. Blocks of paths in a head-to-head pattern having a second type child node or a second type descendent node, such as the pattern 523 and the pattern 524, are released.

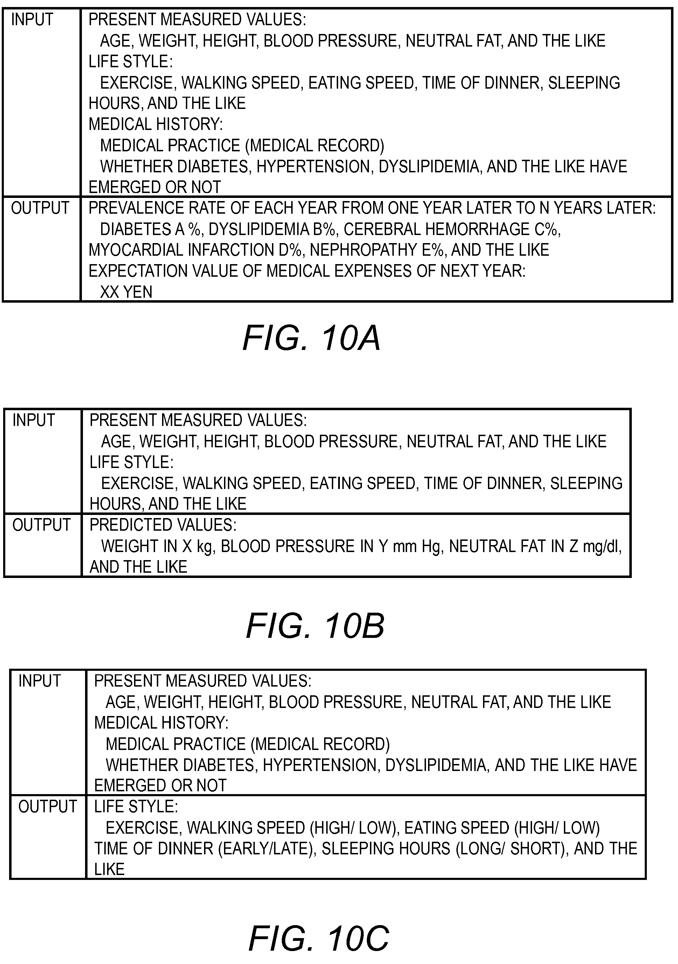

Examples of inputs and outputs of the disease onset prediction apparatus 100 in this embodiment are explained below. FIG. 10A depicts an example of input and output of the disease onset prediction apparatus 100 applied to predicting future disease onset and medical expenses. The disease onset prediction apparatus 100 may receive present measured values, such as a height and a weight, life style, and a medical history as an input and output future disease onset probabilities and an expectation value of medical expenses.

FIG. 10B depicts an example of input and output of the disease onset prediction apparatus 100 applied to predicting future measured values based on life style. The disease onset prediction apparatus 100 may receive present measured values, such as heights and weights, and life style as an input and may output future predicted values, such as a weight and a blood pressure. The disease onset prediction apparatus 100 may output not the predicted values but ranges in which the predicted values are.

FIG. 10C depicts an example of input and output of the disease onset prediction apparatus 100 applied to inferring life style. The disease onset prediction apparatus 100 may receive present measured values, such as heights and weights, and output present life style.

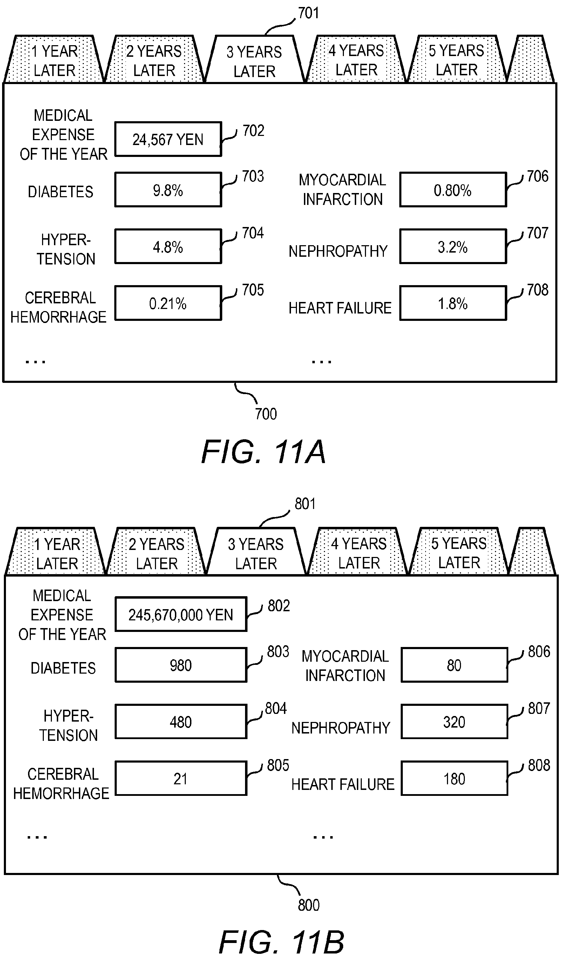

FIG. 11A is an example of a screen showing prediction results of an individual. The prediction result output unit 107 outputs the screen to the output unit 112. A prediction result output screen 700, for example, includes tabs 701 and showing areas 702 to 708. The showing areas 702 to 708 show predicted values of a year designated by one of the tabs 701. The showing area 702 shows an expectation value of medical expenses of the individual for a year. The showing areas 703 to 708 show probability that disease will have been emerged of the individual.

FIG. 11B is an example of a screen showing prediction results of a group including a plurality of persons. A prediction result output screen 800, for example, includes tabs 801 and showing areas 702 to 708. The showing areas 802 to 808 show predicted values of a year designated by one of the tabs 701. The showing area 802 shows an expectation value of medical expenses of the group for a year. The showing areas 803 to 808 show the numbers of patients of disease in the group.

The screens may show not only information about prediction results by stochastic inference but also information about the S/H select table 1100 and information about high priority random variables in which the soft evidence is inputted in due to a restriction on a calculation amount.

As described above, the disease onset prediction apparatus 100 receives medical data about the analysis targets on a certain point and predicts states of a point when one year has passed from the certain point by stochastic inference with a single year model which is a Bayesian network. The disease onset prediction apparatus 100 predicts a two-year-later state by inputting the predicted one-year-after state to the single year model. Thus, the disease onset prediction apparatus 100 can predict N-year chronological change of the medical data by executing recursive prediction N times.

In the recursive prediction, the disease onset prediction apparatus 100 enables accurate prediction with a small calculation amount by selecting whether the soft evidence or the hard evidence is inputted to each input random variable based on feature values of random variables. In particular, the disease onset prediction apparatus 100 can prevents medical states corresponding to the probability distributions whose deviations are great from converging even though the prediction is repeated.

The disease onset prediction apparatus 100 in this embodiment executes prediction with a Bayesian network. However, other graphical models, such as a Markov network, may be used in the prediction instead of the Bayesian network.

This invention is not limited to the above-described embodiments but includes various modifications. The above-described embodiments are explained in details for better understanding of this invention and are not limited to those including all the configurations described above. A part of the configuration of one embodiment may be replaced with that of another embodiment; the configuration of one embodiment may be incorporated to the configuration of another embodiment. A part of the configuration of each embodiment may be added, deleted, or replaced by that of a different configuration.

The above-described configurations, functions, and processors, for all or a part of them, may be implemented by hardware: for example, by designing an integrated circuit. The above-described configurations and functions may be implemented by software, which means that a processor interprets and executes programs providing the functions. The information of programs, tables, and files to implement the functions may be stored in a storage device such as a memory, a hard disk drive, or an SSD (Solid State Drive), or a storage medium such as an IC card, or an SD card.

The drawings shows control lines and information lines as considered necessary for explanations but do not show all control lines or information lines in the products. It can be considered that almost of all components are actually interconnected.

## Claims

1. A chronological change prediction system predicting states of random variables with a graphical model, the chronological change prediction system comprising:
a processor; and
a memory device,
wherein the memory device is configured to store the graphical model,
wherein an input of the graphical model is a first evidence set at a first time,
wherein an output of the graphical model is information about states of the random variables at a second time when a first period has passed from the first time,
wherein the first evidence set includes probability distributions of all or a part of the random variables,
wherein the processor is configured to execute prediction processing with the graphical model, and
wherein, in the prediction processing, the processor is configured to:
obtain the first evidence set;
determine whether to convert each of the probability distributions based on a feature value calculated from each of the probability distributions and/or a feature value calculated from a random variable associated in the graphical model with each of the probability distributions;
convert each probability distribution determined to be converted into a first particular state of a first random variable corresponding to each probability distribution determined to be converted;
create a second evidence set from the first evidence set by replacing each probability distribution determined to be converted and included in the first evidence set with the first particular state;
input the second evidence set to the graphical model; and
output information about states of the random variables of the second time.

2. The chronological change prediction system according to claim 1,
wherein the processor is configured to:
repeat the prediction processing; and
obtain, in second or later prediction processing, information about states of the random variables of the second time outputted in last prediction processing as the first evidence group.

3. The chronological change prediction system according to claim 1,
wherein the feature value calculated from the random variable associated in the graphical model with each of the probability distributions is a number of states of the random variable.

4. The chronological change prediction system according to claim 1,
wherein the feature value calculated from the random variable associated in the graphical model with each of the probability distributions is a value indicating whether the random variable is ordinal or not.

5. The chronological change prediction system according to claim 1,
wherein the feature value calculated from the random variable associated in the graphical model with each of the probability distributions is a deviation of a prior probability distribution of the random variable.

6. The chronological change prediction system according to claim 1,
wherein the feature value calculated from each of the probability distributions is an entropy value of a random variable corresponding to each of the probability distributions.

7. The chronological change prediction system according to claim 1,
wherein, in the prediction processing, the processor is configured to:
select, from the first evidence set, a first probability distribution which is a candidate to be converted based on a feature value calculated from the first probability distribution and/or a feature value calculated from a random variable associated in the graphical model with the first probability distribution;
calculate a calculation amount for, on condition that the first probability distribution is converted into the first particular state, inputting the second evidence set to the graphical model and outputting the information about the states of the random variables of the second time based on association between the random variables and a number of states of a random variable included in the first evidence set and corresponding to a probability distribution which differs from the first probability distribution;
select, when the calculation amount is greater than a predetermined threshold, another probability distribution from the first evidence set as the first probability distribution; and
determine, when the calculation amount is less than or equals to the predetermined threshold, the first probability distribution as the probability distribution determined to be converted, and
wherein the association and the number of states are described in the graphical model.

8. The chronological change prediction system according to claim 1,
wherein, in the prediction processing, the processor is configured to:
determine a method for converting each probability distribution determined to be converted into the first particular state based on a first value, which is described in the graphical model and indicates whether each first random variable is ordinal or not; and
convert, with the determined method, each probability distribution determined to be converted into the first particular state.

9. The chronological change prediction system according to claim 8,
wherein, in the prediction processing, the processor is configured to:
calculate differences between each pair of neighboring possible states of each ordinal first random variable corresponding to the probability distribution determined to be converted; and
determine a method for converting each probability distribution determined to be converted and corresponding to the ordinal first random variable into the first particular state based on the differences.

10. The chronological change prediction system according to claim 1,
wherein the graphical model describes association between the random variables,
wherein the memory device stores association information about association between the random variables which differs from the association described in the graphical model,
wherein, in the prediction processing, the processor is configured to:
receive an identifier of a second random variable included in the association information; and
determine whether to convert each of the probability distributions into the first particular state of the random variable corresponding to each of the probability distributions based on the association in the association information between the random variable corresponding to each of the probability distributions and the second random variable.

11. A method for predicting a state of random variables with a graphical model by a chronological change prediction system,
wherein the chronological change prediction system is configured to store the graphical model,
wherein an input of the graphical model is a first evidence set at a first time,
wherein an output of the graphical model is information about states of the random variables at a second time when a first period has passed from the first time, and
wherein the first evidence set includes probability distributions of all or a part of the random variables, and
the method comprising:
obtaining, by the chronological change prediction system, the first evidence set;
determining, by the chronological change prediction system, whether to convert each of the probability distributions based on a feature value calculated from each of the probability distributions and/or a feature value calculated from a random variable associated in the graphical model with each of the probability distributions;
converting, by the chronological change prediction system, each probability distribution determined to be converted into a first particular state of a first random variable corresponding to each probability distribution determined to be converted;
creating, by the chronological change prediction system, a second evidence set from the first evidence set by replacing each probability distribution determined to be converted and included in the first evidence set with the first particular state;
inputting, by the chronological change prediction system, the second evidence set to the graphical model; and
outputting, by the chronological change prediction system, information about states of the random variables of the second time.

12. A non-transitory computer readable medium storing a program which causes a chronological change prediction system to predict a state of random variables with a graphical model,
wherein the chronological change prediction system is configured to store the graphical model,
wherein an input of the graphical model is a first evidence set at a first time,
wherein an output of the graphical model is information about states of the random variables at a second time when a first period has passed from the first time,
wherein the first evidence set includes probability distributions of all or a part of the random variables, and
wherein the program causes the chronological change prediction system to:
obtain the first evidence set;
determine whether to convert each of the probability distributions based on a feature value calculated from each of the probability distributions and/or a feature value calculated from a random variable associated in the graphical model with each of the probability distributions;
convert each probability distribution determined to be converted into a first particular state of a first random variable corresponding to each probability distribution determined to be converted;
create a second evidence set from the first evidence set by replacing each probability distribution determined to be converted and included in the first evidence set with the first particular state;
input the second evidence set to the graphical model; and
output information about states of the random variables of the second time.
